# EUROPEAN PATENT APPLICATION

(11) **EP 4 617 370 A1**
(43) Date of publication of application: **17.09.2025**
(21) Application number: 23888634.5
(22) Date of filing: 06.11.2023
(51) Int. Cl.: C12P 19/26

(54) **N-ACETYLLACTOSAMINE PRODUCTION METHOD AND N-ACETYLLACTOSAMINE-CONTAINING COMPOSITION**

(30) Priority: 11.11.2022 JP 2022181174
(71) Applicant: Megmilk Snow Brand Co. Ltd., Sapporo-shi, Hokkaido 065-0043 (JP)
(72) Inventor: HIGUCHI Junichi, Sapporo-shi, Hokkaido 065-0043 (JP); YAMAGUCHI Toshiyuki, Sapporo-shi, Hokkaido 065-0043 (JP); NONOYAMA Noriko, Sapporo-shi, Hokkaido 065-0043 (JP); FUKUDOME Hirofumi, Sapporo-shi, Hokkaido 065-0043 (JP); SAKAI Fumihiko, Sapporo-shi, Hokkaido 065-0043 (JP)
(74) Representative: Forstmeyer, Dietmar
(86) International application number: PCT/JP2023/039795
(87) International publication number: WO 2024/101286

(57) **Abstract**

Provided is an N-acetyllactosamine-containing composition production method that achieves a higher rate of conversion from N-acetylglucosamine to N-acetyllactosamine as compared to conventional methods and that is simple but has no effect on the flavor. The production method provided is a method in which N-acetyllactosamine is produced using an enzyme reaction through the action of β-galactosidase on lactose and N-acetylglucosamine, wherein the lactose concentration before the action of β-galactosidase is 1.1 times or more the N-acetylglucosamine concentration in amount-of-substance ratio. An N-acetyllactosamine-containing composition produced by the production method is also provided, wherein the amount-of-substance ratio of N-acetyllactosamine (LacNAc) to N-acetylglucosamine (GlcNAc) (LacNAc/GlcNAc) contained in the composition is 0.26 or more.

## Description

### TECHNICAL FIELD

The present invention relates to a method for producing N-acetyllactosamine and to an N-acetyllactosamine-containing composition.

### BACKGROUND ART

N-acetyllactosamine (hereinafter sometimes referred to simply as LacNAc) is an oligosaccharide consisting of a disaccharide in which galactose (hereinafter sometimes referred to simply as Gal) is linked to N-acetylglucosamine (hereinafter sometimes referred to simply as GlcNAc) via a β-1,4 bond. LacNAc is a biochemically very important oligosaccharide present in the sugar chains of human milk oligosaccharides, lipopolysaccharides, and various glycoproteins and glycolipids. This is also known as a growth factor for bifidobacteria which is a type of enteric bacteria. This is also a useful substance as a functional food ingredient.

The chemical synthesis procedure and synthesis method using Gal transferase with uridine diphosphate galactose (UDP-Gal) and GlcNAc as substrates are known as a method for producing LacNAc. However, the chemical synthesis procedure requires the use of harmful reagents and solvents that cannot be used in food. In the synthesis method using Gal glycosyltransferase, the substrates UDP-Gal and Gal transferase are very expensive. Hence, these synthesis methods cannot be used for industrial production of LacNAc intended for use in food. On the other hand, a method utilizing the glycosyltransferase activity of β-galactosidase is known as a simple method for synthesizing LacNAc. The synthesis method is a method in which Gal residues of lactose and p-nitrophenyl-galactoside(PNP-Gal) are transferred to GlcNAc.

Patent Documents 1, 2, and 3 disclose methods for preparing LacNAc using β-galactosidase derived from the genus Diplococcus pneumoniae bacteria, *Bifidobacterium,* and *Bacillus* bacteria, respectively. In the methods, the PNP-Gal is used as a Gal donor. However, since the PNP-Gal is a synthetic substrate that cannot be used in food, LacNAc prepared by the methods cannot be applied to food. A method for preparing a composition containing a large amount of LacNAc requires a step of concentrating LacNAc after preparing LacNAc using β-galactosidase. However, the preparation method has too high a producing cost to be preferred as a method for preparing a composition for food. Thus, a method allowing LacNAc to be used in food without the step of concentrating LacNAc after its preparation is preferable. For adding LacNAc to food without concentrating it after its preparation, the Gal donor is desirably lactose which is an inexpensive and safe sugar. As the Gal acceptor GlcNAc, high-purity GlcNAc usable as food is commercially available, which is produced using crustaceans such as shrimp and crabs and certain type of plants as raw materials. However, GlcNAc commercially available for food use is very expensive compared to lactose. Hence, LacNAc can be produced at low cost if a more efficient method of converting GlcNAc to LacNAc can be found. Namely, in order to use LacNAc in food at low cost without undergoing the complicated concentration step, (1) using inexpensive and safe lactose as the Gal donor, and (2) finding conditions for an enzyme reaction that provides a high conversion rate of GlcNAc, used as the Gal acceptor, to LacNAc are required.

Patent Document 4 discloses a method for preparing LacNAc by allowing β-galactosidase derived from *Bacillus circulans* to act on a solution containing lactose and GlcNAc. Patent Document 4 discloses preparing LacNAc by setting an amount-of-substance ratio of lactose to GlcNAc (lactose/GlcNAc ratio) to 0.25 to 0.5. Patent Document 4 discloses that the conversion rate of GlcNAc to LacNAc is at most about 15% and that an amount-of-substance ratio of N-acetyllactosamine (LacNAc) to N-acetylglucosamine (GlcNAc) (LacNAc/GlcNAc) is at most 0.18 (Example (1)).

Patent Document 5 discloses a method for producing LacNAc by allowing β-galactosidase to act on lactose and GlcNAc. A production method is disclosed in which LacNAc is produced in high yield by coexisting β-galactosidase and glucose oxidase as enzymes. Another description is that the conversion rate of GlcNAc to LacNAc reaches up to 21% using the production method (Table 1). However, the addition of preparations containing an enzyme such as glucose oxidase is undesirable because it increases cost and degrades the flavor of composition.

### PATENT LITERATURE

Patent Document 1: JP H6,335,395 A
Patent Document 2: JP H10,23,898 A
Patent Document 3: JP 6,771,756 B
Patent Document 4: JP 2,819,312 B
Patent Document 5: JP 2001,292,791 A

### SUMMERY OF INVENTION

### TECHNICAL PROBLEM

A task of the present invention is to provide a method for producing an N-acetyllactosamine-containing composition using lactose, N-acetylglucosamine, and β-galactosidase, wherein a conversion rate of N-acetylglucosamine to N-acetyllactosamine is higher than conventional methods. The production method is one which is simple and does not affect the flavor of composition. Another problem of the present invention is to provide an N-acetyllactosamine composition in which an amount-of-substance ratio of N-acetyllactosamine to N-acetylglucosamine (LacNAc/GlcNAc) is 0.26 or more by using such a method.

### SOLUTION TO PROBLEM

As a result of advanced intensive study to solve the above problems, the inventors found that the above problems can be solved by making the lactose concentration before an enzyme reaction 1.1 times or more the N-acetylglucosamine concentration in terms of amount-of-substance, and completed the present invention. That is, the present invention has the following configurations.
<1> A method for producing N-acetyllactosamine, comprising bringing a solution containing lactose and N-acetylglucosamine into contact with β-galactosidase, wherein the lactose concentration in the solution before contact with β-galactosidase is 1.1 times or more the N-acetylglucosamine concentration in amount-of-substance ratio.
<2> The production method according to <1>, wherein only β-galactosidase is allowed to act as an enzyme.
<3> The production method according to <1> or <2>, wherein the conversion rate of N-acetylglucosamine to N-acetyllactosamine by an enzyme reaction is 20% or more in terms of amount-of- substance.
<4> The production method according to <1> or <2>, wherein the β-galactosidase is one derived from genus *Bacillus* or genus *Bifidobacterium.*
<5> A method for producing N-acetyllactosamine, comprising bringing a solution containing lactose and N-acetylglucosamine into contact with β-galactosidase, wherein the β-galactosidase is one derived from genus *Bifidobacterium.*
<6> The production method according to <5>, wherein only β-galactosidase is allowed to act as an enzyme.
<7> The production method according to <5> or <6>, wherein the rate of conversion of N-acetylglucosamine to N-acetyllactosamine by an enzyme reaction is 20% or more in terms of amount-of- substance.
<8> A composition comprising N-acetyllactosamine produced by the method according to any one of <1>, <2>, <5>, or <6>, wherein the amount-of-substance ratio of N-acetyllactosamine (LacNAc) to N-acetylglucosamine (GlcNAc) contained in the composition (LacNAc/GlcNAc) is 0.26 or more.
<9> A composition comprising N-acetyllactosamine produced by bringing a solution containing lactose and N-acetylglucosamine into contact with only β-galactosidase as an enzyme, wherein the amount-of-substance ratio of N-acetyllactosamine (LacNA c) to N-acetylglucosamine (GlcNAc) (LacNAc/GlcNAc) is 0.26 or more.

### ADVANTAGEOUS EFFECTS OF INVENTION

According to the present invention, a method for producing N-acetyllactosamine with a high conversion rate of N-acetylglucosamine to N-acetyllactosamine can be provided by making a lactose concentration before an enzyme reaction 1.1 times or more an N-acetylglucosamine concentration in terms of amount-of-substance. In this production method, N-acetylglucosamine can be efficiently converted to N-acetyllactosamine even by using only β-galactosidase as an enzyme. Hence, this production method is one that is inexpensive and has no adverse effect on the flavor.

In this production method, conversion efficiency of N-acetylglucosamine to N-acetyllactosamine is high, thereby providing at low cost an N-acetyllactosamine-containing composition usable in food without going through a complicated concentration step after the enzyme reaction.

### DESCRIPTION OF EMBODIMENT

### (Production Method)

The present invention is a method for producing N-acetyllactosamine using an enzyme reaction by the action of β-galactosidase on lactose and N-acetylglucosamine, comprising a step of contacting a solution containing lactose and N-acetylglucosamine with β-galactosidase,wherein a lactose concentration in said solution before the action of β-galactosidase is 1.1 times or more an N-acetylglucosamine concentration in an amount-of-substance ratio. Typically, said production method is a method in which β-galactosidase is added to a solution containing lactose and N-acetylglucosamine to produce N-acetyllactosamine. β-galactosidase,an enzyme with galactosyltransferase activity, acts on the substrates, lactose and N-acetylglucosamine. In this production method, N-acetyllactosamine can be produced efficiently by making the lactose concentration in above-mentioned solution before the action of β-galactosidase 1.1 times or more the N-acetylglucosamine concentration in the amount-of-substance ratio.

As the enzyme, other enzymes can be added as long as they do not inhibit the galactosyltransferase activity of β-galactosidase. For example, adding such as glucose oxidase can remove glucose, which is a by-product of an enzymatic synthesis reaction of N-acetyllactosamine using lactose and N-acetylglucosamine as the substrates. However, it is desirable to use β-galactosidase only as the enzyme, in view of effects on flavor and cost reduction. According to the production method of the present invention, a conversion rate of N-acetylglucosamine to N-acetyllactosamine can achieve 20% or more even by using only β-galactosidase as the enzyme.

### (β-galactosidase)

In the production method of the invention, β-galactosidase refers to the enzyme with an action of catalyzing a reaction which transfers galactose residues from lactose to N-acetylglucosamine, and is not particularly limited as long as they have such an action. As such an enzyme, in addition to purified enzymes, such as resting bacterial cells, dried bacterial cells, and crushed bacterial cells that have the action of the enzyme may also be used.

Specific examples of the enzyme may include purified or partially purified β-galactosidase enzymes derived from bacteria belonging to the genus *Bacillus* such as *Bacillus circulans,* the genus *Rhodotorula* such as *Rhodotorula minuta* and *Rhodotorula lactosa,* the genus *Streptococcus* such as *Streptococcus pneumoniae* and Streptococcus sp. 6646k strain, the genus *Sterigmatomyces* such as *Sterigmatomyces circulans,* and the genus *Bifidobacterium.* Substances containing the enzyme (resting bacterial cells, dried bacterial cells, crushed bacterial cells, etc.) may also be preferably used. N-acetyllactosamine is a disaccharide in which D-galactose and N-acetylglucosamine are linked via a β-1,4-glycosidic bond. Hence, β-galactosidase with high specificity for the β-1,4-glycosidic bonds is preferable. In particular, purified or partially purified β-galactosidase enzymes derived from the genus *Bacillus* or *Bifidobacterium,* or the substances containing the enzymes (resting bacterial cells, dried bacterial cells, crushed bacterial cells, etc.) are more preferable.

A concentration of β-galactosidase can be appropriately set by those skilled in the art so that the above-mentioned enzyme reaction proceeds suitably. In a case that β-galactosidase is used, the final concentration is preferably 0.01 U/ml to 100 U/ml, and is preferably 0.1 U/ml to 10 U/ml. Here, the enzyme activity of 1 U of β-galactosidase is defined as the amount of enzyme that releases 1 µmol of glucose per minute in a case that the enzyme is added to a lactose solution (pH 6.5) to allow them react at 50 °C for 10 minutes.

The above-mentioned enzyme reaction in the production method of the present invention is, in a solution (e.g., aqueous solution, buffer solution) containing lactose and N-acetylglucosamine, a reaction in which β-galactosidase is made to act on these substrates to produce N-acetyllactosamine. As long as this enzyme reaction proceeds, the reaction conditions and the like are not limited. The enzyme reaction mentioned above is preferably conducted in a buffer solution at an optimal pH and temperature for β-galactosidase. For example, in a case that β-galactosidase derived from the genus *Bacillus* is used, the enzyme reaction is preferably carried out in a buffer where the pH can be retained under the condition of 4 to 9 in pH and 5 to 70°C. When β-galactosidase derived from the genus *Bifidobacterium* is used, the enzyme reaction is preferably carried out in a buffer where the pH can be retained under the condition of 4 to 9 in pH and 5 to 70°C.

Lactose and N-acetylglucosamine, which are the substrates for the above enzyme reaction, are usually used as an aqueous solution or buffer solution containing lactose and N-acetylglucosamine. The contents and content ratios of the respective substances, etc. are not particularly limited as long as they are not unfavorable to the above enzyme reaction. Lactose is preferably used at a concentration of about 0.5 to 1.5 M, more preferably about 1 M. N-acetylglucosamine is preferably used at a concentration of about 0.05 to 1 M, more preferably about 0.5 M. The lactose concentration in the solution before the start of enzyme reaction may be 1.1 times or more the N-acetylglucosamine concentration in amount-of-substance ratio, preferably about 1.1 to 52 times, and more preferably about 1.1 to 5 times. The amount-of-substance ratio can be determined by dividing the lactose concentration (M) of the substrate before the start of enzyme reaction by the N-acetylglucosamine concentration (M) of the substrate before the start of enzyme reaction. If the amount-of-substance ratio is 1.1 times or more, a conversion rate of N-acetylglucosamine to N-acetyllactosamine reaches 20% or more, regardless of types of β-galactosidases.

The higher an amount-of-substance ratio of lactose/N-acetylglucosamine is, the higher the conversion rate of N-acetylglucosamine to N-acetyllactosamine becomes. Meanwhile, the higher the amount-of-substance ratio of lactose/N-acetylglucosamine is, the lower the amount-of-substance of N-acetyllactosamine produced becomes. In other words, when the amount-of-substance ratio of lactose/N-acetylglucosamine is low, a production amount of N-acetyllactosamine increases, but the conversion rate of N-acetylglucosamine to N-acetyllactosamine decreases, resulting in a waste of expensive N-acetylglucosamines.

Therefore, in order to achieve the conversion rate of N-acetylglucosamine to N-acetyllactosamine of 20% or more and a certain or greater amount-of-substance of N-acetyllactosamine, the lactose concentration before the start of enzyme reaction is desirably adjusted to be 1.1 times or more the N-acetylglucosamine concentration before the enzyme reaction in terms of the amount-of-substance.

The conversion rate of N-acetylglucosamine to N-acetyllactosamine after the enzyme reaction is desirably high, and is preferably 20% or more in terms of the amount-of-substance, more preferably 21% or more, and even more preferably 25% or more. The conversion rate can be calculated by dividing the N-acetyllactosamine concentration (M) after the end of enzyme reaction by the N-acetylglucosamine concentration (M) of the substrate before the start of enzyme reaction, multiplying the resulting value by 100.

### (N-acetyllactosamine-Containing Composition)

The N-acetyllactosamine-containing composition of the invention is a composition in which the amount-of-substance ratio of N-acetyllactosamine to N-acetylglucosamine (LacNAc/GlcNAc) is 0.26 or more. The composition can be produced by a method including a step of allowing only β-galactosidase to act as the enzyme in the solution containing lactose and N-acetylglucosamine. In particular, said composition can be produced by allowing β-galactosidase to act in a solution containing lactose and N-acetylglucosamine. The concentration of lactose in said solution is 1.1 times or more the concentration of N-acetylglucosamine by amount-of-substance ratio. In a case that only β-galactosidase is allowed to act as the enzyme on lactose and N-acetylglucosamine, the expected benefit is that no other unnecessary production is produced, thereby having little effect on the flavor.

The above amount-of-substance ratio (LacNAc/GlcNAc) is the value obtained by dividing a LacNAc concentration (M) by a GlcNAc concentration (M) after the end of enzyme reaction. Here, the GlcNAc concentration (M) after the end of enzyme reaction can also be substituted with the value obtained by subtracting the LacNAc concentration (M) after the end of enzyme reaction from the GlcNAc concentration (M) of the substrate before the start of enzyme reaction.

### EXAMPLES

### [Example 1]

### 1. Test Method

N-acetyllactosamine (LacNAc) was prepared by adding β-galactosidases derived from the genus *Bifidobacterium* (Novozymes Japan Ltd.: Saphera Fiber) or β-galactosidases derived from the genus *Bacillus* bacteria (Amano Enzyme Inc.: Biolacta FN5) to 1 mL of 50 mM acetate buffer (pH 6.0) in which lactose and N-acetyllactosamine (GlcNAc) were dissolved under the nine conditions shown in Table 1. The reaction solution was boiled for 5 minutes to stop the reaction. Subsequently, lactose and N-acetyllactosamine (LacNAc) in the solution after the end of enzyme reaction were respectively quantified by ion chromatography under the following detection conditions.

A conversion rate to N-acetyllactosamine (LacNAc) was calculated using the following formula (1). An amount-of-substance ratio of LacNAc/GlcNAc was calculated using the following formula (2). The results are shown in Table 1. Conversion rate to LacNAc (%) =LacNAc concentration in solution after end of enzyme reaction (M)÷GlcNAc concentration of substrate (M)×100 Amount-of-substance ratio of LacNAc/GlcNAc=LacNAc concentration in solution after end of enzyme reaction (M)÷ (GlcNAc concentration of substrate (M)-LacNAc concentration in solution after end of enzyme reaction (M))

### <Ion chromatography detection conditions>

Column: CarboPac PA1 (made by Thermo Fisher Scientific Inc.)
Mobile phase: 100 mM NaOH
Flow rate: 1 mL/min
Temperature: 25°C
Detector: electrochemical detector
Sample: 10 uL

**[Table 1]**

| Relationship in LacNac preparation between substrate concentration and production amount of LacNAc | | | | | | | | | |
|---|---|---|---|---|---|---|---|---|---|
| | Reaction condition | | | Substrate concentration | | | Product concentration | | |
| *β* -galactosidase | Enzyme temperature | Temperature | Time | Lactose | GlcNAc | Lactose/ GlcNAc | LacNAc | Conversion rate to LacNAc | LacNAc/ GlcNAc |
| | U/mL | °C | h | M | M | amount-of-substance ratio | M | | amount-of-substance ratio |
| *β* -galactosidase derived from the genus *Bacillus* | 5.0 | 50 | | 1.02 | 0.23 | 4.5 | 0.06 | 25% | 0.35 |
| | 5.0 | 50 | 4 2 | 0.88 | 0.45 | 1.9 | 0.10 | 21% | 0.27 |
| | 5.0 | 50 | 4 | 0.73 | 0.68 | 1.1 | 0.14 | 20% | 0.26 |
| | 5.0 | 50 | 4 | 0.58 | 0.90 | 0.65 | 0.15 | 17% | 0.21 |
| *β* -galactosidase derived from the genus *Bifidobacterium* | 2.2 | 60 | 1 | 1.17 | 0.02 | 51.7 | 0.01 | 34% | 0.57 |
| | 5.0 | 50 | 1 | 1.02 | 0.23 | 4.5 | 0.08 | 33% | 0.50 |
| | 5.0 | 50 | 1 | 0.88 | 0.45 | 1.9 | 0.12 | 26% | 0.35 |
| | 5.0 | 50 | 1 | 0.73 | 0.68 | 1.1 | 0.14 | 20% | 0.26 |
| | 5.0 | 50 | 4 | 0.58 | 0.90 | 0.65 | 0.16 | 18% | 0.23 |

### 2. Results and Discussion

Table 1 shows that the higher the amount-of-substance ratio of lactose/GlcNAc is, the higher the conversion rate of N-acetylglucosamine to N-acetyllactosamine becomes. If the lactose concentration before the start of enzyme reaction was 1.1 times or more the N-acetylglucosamine concentration in terms of amount-of-substance, the conversion rate of N-acetylglucosamine to N-acetyllactosamine reached 20% or more, regardless of the type of enzyme. The higher the proportion of N-acetylglucosamine (the lower the amount-of-substance ratio of lactose/GlcNAc is) is, the higher the production amount of N-acetyllactosamine is. However, the higher the proportion of N-acetylglucosamine is, the lower the conversion rate of N-acetylglucosamine to N-acetyllactosamine is, resulting in a waste of expensive N-acetylglucosamine.

Thus, as shown above, the lactose concentration before the enzyme reaction needs to be adjusted to 1.1 times or more the N-acetylglucosamine concentration in terms of amount-of- substance in order to convert expensive N-acetylglucosamine to N-acetyllactosamine at proportion of 20% or more.

The amount-of-substance ratio of N-acetyllactosamine and N-acetylglucosamine (LacNAc/GlcNAc) contained in an N-acetyllactosamine-containing composition prepared by this production method was 0.26 or more.

### [Example 2]

### 1. Test Method

A solution was prepared to which 17.6% powdered skim milk (corresponding to 8.4% of the lactose concentration) and N-acetylglucosamine material (Yaizu Suisan Kagaku Kogyo Co., Ltd.: Marine Sweet YM40) corresponding to 4.3% of the N-acetylglucosamine concentration are added. β-galactosidase derived from the genus *Bacillus* bacteria (Amano Enzyme Inc.: Biolacta FN5) was added to the solution at a concentration of 5.0 U/mL and reacted at 7°C for 20 hours. Thereafter, the reaction solution was boiled for 5 minutes to stop the reaction. The production amount of LacNAc was then measured by ion chromatography under the conditions of Example 1.

### 2. Results

The production amount of N-acetyllactosamine was 1.6% (the conversion rate from GlcNAc was 21%), and the amount-of-substance ratio of N-acetyllactosamine to N-acetylglucosamine (LacNAc/GlcNAc) in the resulting N-acetyllactosamine-containing composition was 0.52. The amount-of-substance ratio of lactose/GlcNAc of the substrates of this example was 1.3.

### [Example 3]

### 1. Test Method

An aqueous solution with a total weight of 100 kg was prepared in which 32 kg of desalted whey powder (25 kg in terms of lactose) and N-acetylglucosamine material (Yaizu Suisan Kagaku Kogyo Co., Ltd.: Marine Sweet YM40) in an amount of 6.3 kg in terms of N-acetylglucosamine were dissolved. 500,000 U of β-galactosidase derived from the genus *Bifidobacterium* (Novozymes Japan Ltd.: Saphera Fiber) was added to the aqueous solution, and the mixture was reacted at 50 °C for 1 hour.
Thereafter, the reaction solution was heated at 95°C for 10 minutes to stop the reaction.
Following that, the production amount of LacNAc was measured by ion chromatography as in Example 1.

### 2. Results

The production amount of N-acetyllactosamine was 3.0 kg (the conversion rate from GlcNA was 27%). The amount-of-substance ratio of N-acetyllactosamine to N-acetylglucosamine (LacNAc/GlcNAc) in the resulting N-acetyllactosamine-containing composition was 0.38. The lactose/GlcNAc ratio of the substrate in this example was 2.6.

### INDUSTRIAL APPLICABILITY

According to the present invention, there can be provided a method for producing N-acetyllactosamine with a high conversion rate of N-acetylglucosamine to N-acetyllactosamine. With use of the production method of the present invention, expensive N-acetylglucosamine used as a Gal acceptor can be converted efficiently to N-acetyllactosamine using inexpensive and safe lactose as a Gal donor. Therefore, N-acetyllactosamine usable in food can be produced at a lower cost than before without going through a complicated concentration process.

## Claims

1. A method for producing N-acetyllactosamine, comprising bringing a solution containing lactose and N-acetylglucosamine into contact with β-galactosidase, wherein the lactose concentration in the solution before contact with β-galactosidase is 1.1 times or more the N-acetylglucosamine concentration in amount-of-substance ratio.

2. The production method according to claim 1, wherein only β-galactosidase is allowed to act as an enzyme.

3. The production method according to claim 1 or 2, wherein the conversion rate of N-acetylglucosamine to N-acetyllactosamine by an enzyme reaction is 20% or more in terms of amount-of- substance.

4. The production method according to claim 1 or 2, wherein the β-galactosidase is one derived from genus *Bacillus* or genus *Bifidobacterium.*

5. A method for producing N-acetyllactosamine, comprising bringing a solution containing lactose and N-acetylglucosamine into contact with β-galactosidase, wherein the β-galactosidase is one derived from genus *Bifidobacterium.*

6. The production method according to claim 5, wherein only β-galactosidase is allowed to act as an enzyme.

7. The production method according to claim 5 or 6, wherein the rate of conversion of N-acetylglucosamine to N-acetyllactosamine by an enzyme reaction is 20% or more in terms of amount-of- substance.

8. A composition that contains N-acetyllactosamine produced by the method according to any one of claim 1, 2, 5, or 6, wherein the amount-of-substance ratio of N-acetyllactosamine (LacNAc) to N-acetylglucosamine (GlcNAc) contained in the composition (LacNAc/GlcNAc) is 0.26 or more.

9. A composition that contains N-acetyllactosamine produced by bringing a solution containing lactose and N-acetylglucosamine into contact with only β-galactosidase as an enzyme, wherein the amount-of-substance ratio of N-acetyllactosamine (LacNAc) to N-acetylglucosamine (GlcNAc) (LacNAc/GlcNAc) is 0.26 or more.
